# FASCICULE DE BREVET EUROPEEN

(11) **EP 3 849 658 B1**
(45) Date de publication et mention de la délivrance du brevet: **25.10.2023**
(21) Numéro de dépôt: 19765503.8
(22) Date de dépôt: 12.09.2019
(51) Int. Cl.: A61N 5/06, A61N 5/067

(54) **SYSTÈME DE LASER IMPULSIONNEL DESTINÉ AUX TRAITEMENTS DERMATOLOGIQUES**
GEPULSTES LASERSYSTEM FÜR DERMATOLOGISCHE BEHANDLUNGEN
PULSED LASER SYSTEM FOR DERMATOLOGICAL TREATMENTS

(30) Priorité: 13.09.2018 FR 1858247
(43) Date de publication de la demande: 21.07.2021
(73) Titulaire: Irisiome, 33600 Pessac (FR)
(72) Inventeur: ROYON, Romain, 40100 DAX (FR); DUBRASQUET, Romain, 40630 SABRES (FR)
(74) Mandataire: Jacobacci Coralis Harle
(86) Numéro de dépôt international: PCT/EP2019/074342
(87) Numéro de publication internationale: WO 2020/053330

(56) Documents cités:
- FR-A1- 2 924 597
- FR-A1- 2 939 284

## Description

### DOMAINE TECHNIQUE AUQUEL SE RAPPORTE L'INVENTION

La présente invention concerne de manière générale le domaine des systèmes lasers.

Elle concerne plus particulièrement un système de laser à impulsions destiné aux traitements dermatologiques appliqués au corps humain ou animal.

### ARRIERE-PLAN TECHNOLOGIQUE

L'utilisation d'impulsions laser trouve de nombreuses applications dans les domaines scientifique, industriel ou médical.

En dermatologie, différents lasers sont utilisés pour de nombreuses applications comme l'épilation, le rajeunissement de peau, la suppression de tache pigmentaire ou de tatouage, aussi appelé le détatouage.

La plupart des lasers actuellement disponibles adaptés pour les applications en dermatologie génèrent des impulsions longues et énergétiques qui induisent un effet thermique de photo-ablation. Dans le présent document, on entend par impulsions longues, des impulsions laser de durée supérieure à plusieurs centaines de picosecondes (ps), généralement des impulsions de durée sub-nanoseconde, nanoseconde ou microseconde. On entend par impulsion énergétique, une impulsion laser délivrant une énergie au moins de l'ordre du Joule. Les lasers délivrant ces impulsions longues et énergétiques fonctionnent à une faible fréquence de répétition des impulsions, généralement inférieure à 10 hertz (Hz), par exemple comprise entre 1 Hz et 10 Hz.

Dans la plupart des systèmes laser de dermatologie connus, l'utilisateur, par exemple un dermatologue, tient une pièce manuelle qui lui permet d'appliquer le faisceau d'impulsions laser longues, énergétiques et à une faible fréquence de répétition sur une zone de la peau à traiter. Le faisceau laser est généralement étendu sur une zone circulaire ayant un diamètre de plusieurs millimètres. Lorsque le faisceau laser couvre toute la surface à traiter, il n'est pas nécessaire de déplacer le faisceau laser. Dans certains cas, l'utilisateur déplace la pièce manuelle pas par pas pour traiter une autre zone de la peau. De plus, la taille du spot limite la résolution spatiale de ces traitements laser.

Toutefois, l'utilisation d'un tel système laser n'est pas sans risque en particulier d'ablation et/ou d'échauffement local pouvant produire une sensation de brûlure pour le patient. De plus, l'efficacité de certains traitements lasers en dermatologie est imparfaite, ce qui nécessite plusieurs séances de traitement laser, par exemple pour le détatouage. Une focalisation du spot pour tenter d'augmenter la résolution spatiale impliquerait une augmentation de l'intensité laser, ce qui augmenterait encore les risques d'échauffement.

Enfin, compte tenu de la variété des traitements et des types de peau, il est souvent nécessaire de disposer de plusieurs systèmes laser spécifiques, chaque système étant dédié à une application. Le document FR 2 939 284 divulgue un système pour traiter des cheveux ou cils. Les impulsions laser ont la durée de 10⁻¹⁵ - 10⁻⁷ s, la fréquence des impulsions est entre 100 Hz - 10⁵ Hz, et la puissance des impulsions - entre 10⁶ - 10¹² W. La densité d'énergie est 10-³ J/cm². Il y a un système de balayage.

Il est souhaitable de développer un système laser pour application dermatologique à la fois plus efficace, moins invasif, ayant une meilleure résolution spatiale tout en réduisant les risques d'échauffement ou de brûlure pour le patient. Il est souhaitable de développer un système laser pour application de traitement dermatologique plus compact et moins coûteux que les systèmes laser de l'art antérieur.

### OBJET DE L'INVENTION

Afin de remédier à l'inconvénient précité de l'état de la technique, la présente invention propose un système laser impulsionnel pour traitement dermatologique, le système laser impulsionnel comprenant une source lumineuse adaptée pour émettre un faisceau d'impulsions lumineuses et un système amplificateur optique adapté pour recevoir le faisceau d'impulsions lumineuses et pour générer un faisceau d'impulsions laser à une première fréquence de répétition.

Plus particulièrement, on propose selon l'invention un système laser impulsionnel pour traitement dermatologique dans lequel la durée (d) d'une impulsion laser est comprise entre 100 femtoseconde et 100 picosecondes, la première fréquence de répétition est comprise entre 1 kHz et 10 GHz, chaque impulsion laser ayant une quantité d'énergie inférieure ou égale à 10 microjoule, et le système laser impulsionnel comporte en outre des moyens de modulation temporelle du faisceau d'impulsions laser ces moyens de modulation temporelle étant adaptés pour réduire la densité d'énergie déposée sur une surface à traiter et pour générer une densité d'énergie comprise entre 0.0001 J/cm² et 0.01 J/cm².

Les moyens de modulation temporelle du faisceau d'impulsions laser comprennent un modulateur acousto-optique et un générateur électrique, le modulateur acousto-optique étant disposé en sortie de système amplificateur optique, le générateur électrique étant adapté pour générer un signal radio-fréquence appliqué aux électrodes du modulateur acousto-optique, le signal radio-fréquence étant adapté pour que le modulateur acousto-optique sélectionne un paquet d'impulsions laser.

Alternativement, le système amplificateur optique comporte un dispositif de pompage optique et une source de courant-tension et les moyens de modulation temporelle du faisceau d'impulsions laser comprennent un générateur électrique adapté pour générer un signal radio-fréquence appliqué à la source de courant-tension du dispositif de pompage optique, le signal radio-fréquence étant adapté pour que le système amplificateur optique génère un paquet d'impulsions laser.

De façon particulièrement avantageuse, le générateur électrique est en outre adapté pour moduler temporellement le signal radio-fréquence de manière à moduler en intensité les impulsions laser d'un paquet d'impulsions laser.

De préférence, un paquet d'impulsions laser comporte un nombre N d'impulsions laser compris entre 100 et 1000000.

De préférence , le système comprend en outre les moyens de modulation spatiale du faisceau d'impulsions laser comprennent un dispositif de balayage de faisceau, le dispositif de balayage de faisceau étant adapté pour déplacer le faisceau d'impulsions laser sur une région d'intérêt de la surface à traiter de manière à limiter la densité d'énergie déposée dans la région d'intérêt.

De façon particulièrement avantageuse, le dispositif de balayage de faisceau est adapté pour déplacer le faisceau laser suivant deux axes transverses.

De préférence, la vitesse de déplacement du faisceau lumineux dans la région d'intérêt est comprise entre 0,1m/s et 10 m/s.

Selon un autre aspect particulier et avantageux, le système laser comporte en outre un système optique de focalisation, le système optique de focalisation est adapté pour focaliser le faisceau laser sur un spot ayant un diamètre inférieur à environ 20 mm.

D'autres caractéristiques non limitatives et avantageuses du système laser impulsionnel pour traitement dermatologique conforme à l'invention, prises individuellement ou selon toutes les combinaisons techniquement possibles, sont les suivantes :
- la source lumineuse et le système amplificateur optique sont adaptés pour émettre le faisceau d'impulsions laser à une longueur d'onde comprise entre 480 nm et 10600 nm.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement en fonction du temps l'intensité lumineuse délivrée par un laser à haute cadence et impulsions longues ;
- la figure 2 représente schématiquement un système laser selon un premier mode de réalisation ;
- la figure 3 représente schématiquement un système laser selon un deuxième mode de réalisation ;
- la figure 4 représente schématiquement en fonction du temps un premier exemple d'intensité lumineuse délivrée par un laser selon le premier ou deuxième mode de réalisation ;
- la figure 5 représente schématiquement en fonction du temps un deuxième exemple d'intensité lumineuse délivrée par un laser selon le premier ou deuxième mode de réalisation ;
- la figure 6 représente schématiquement en fonction du temps un troisième exemple d'intensité lumineuse délivrée par un laser selon le premier ou deuxième mode de réalisation ;
- la figure 7 représente schématiquement un système laser selon un troisième mode de réalisation ;
- la figure 8 représente schématiquement le fonctionnement d'un laser selon le troisième mode de réalisation.

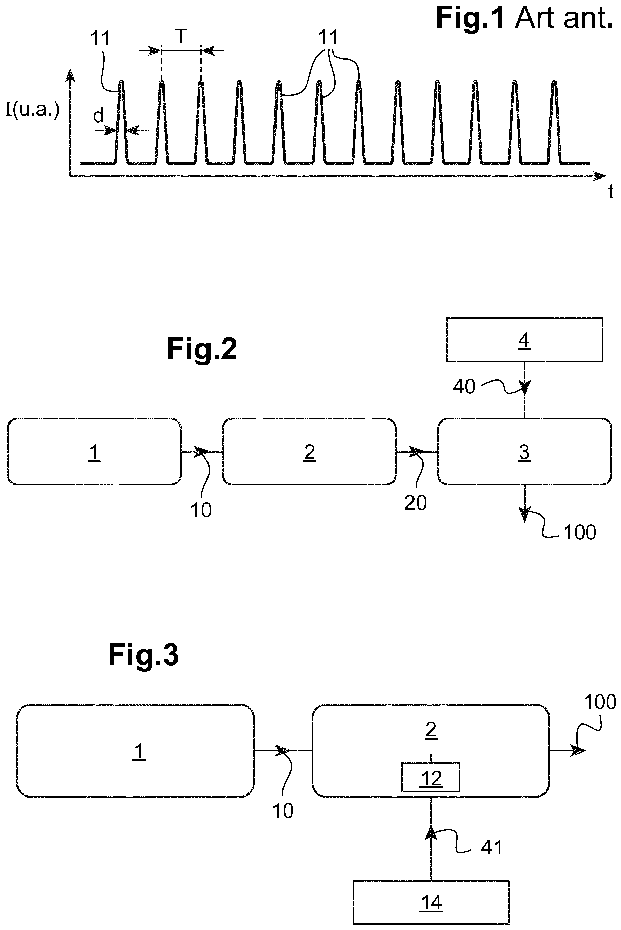

Sur la figure 1, on a représenté en fonction du temps l'intensité lumineuse délivrée par un système laser générant des impulsions longues selon l'art antérieur. On note d la durée d'une impulsion laser 11. Pour des impulsions longues, cette durée d est supérieure à 100 picosecondes (ps), généralement de l'ordre de la nanoseconde ou microseconde. On note T la période de répétition des impulsions laser 11. La période de répétition T est égale à l'inverse de la fréquence de répétition F. La période de répétition T des impulsions laser 11 longues est généralement comprise entre 10⁻¹ s. et 1 s. Autrement dit, la fréquence de répétition F des impulsions laser 11 est comprise entre 1Hz et 10 Hz. Un tel système laser qui génère des impulsions laser 11 longues à une fréquence de répétition F, délivre une énergie de l'ordre du millijoule ou du joule par impulsion. Le faisceau laser est incident sur une zone généralement circulaire étendue ayant un diamètre de plusieurs millimètres sur la surface à traiter. Dans les applications classiques de détatouage, le diamètre du faisceau laser est généralement de l'ordre de 5 mm. Dans les applications classiques pour de l'épilation, le diamètre du faisceau laser est généralement de l'ordre 18 mm.

Un tel système laser génère un dépôt d'une quantité d'énergie par unité de surface pour un temps donné. Ce dépôt d'énergie est compris entre 0.1 J/cm² et 100 J/cm² et généralement de l'ordre de 1 J/cm² par impulsion laser ayant une durée d'impulsion allant de ~500 ps jusqu'à plusieurs nanosecondes. La durée du traitement d'une zone à traiter est généralement comprise entre 5 minutes jusqu'à 60 min. Différents phénomènes physiques peuvent être mis en jeu dans cette gamme de dépôt d'énergie. Pour des impulsions laser 11 ayant une durée supérieure à la nanoseconde (10⁻⁹ s.), on considère que l'absorption du faisceau d'impulsions laser est linéaire et induit une photo-ablation des tissus. Pour des impulsions laser 11 ayant une durée inférieure à la nanoseconde (10⁻⁹ s.) et une densité d'énergie de l'ordre de 1 J/cm², on considère que l'absorption du faisceau d'impulsions laser est non-linéaire et induit une photo-disruption des tissus.

La présente divulgation propose différents systèmes laser qui utilisent des impulsions laser brèves ou ultra-brèves, ayant une durée d comprise entre environ 100 femtosecondes et 100 picosecondes et de préférence inférieure à 10 ps. Selon la présente divulgation, le système laser génère ces impulsions laser brèves ou ultra-brèves à une première fréquence de répétition F₁ élevée, comprise entre 1 kHz et 10 GHz. Autrement dit, les impulsions laser brèves ou ultra-brèves ont une première période de répétition T₁ comprise entre 100×10⁻¹⁵ s et 100 ×10⁻⁹ s. Chaque impulsion lumineuse a une quantité d'énergie inférieure ou égale à 100 microjoules. De plus, le système laser comporte des moyens de modulation temporelle du faisceau laser ces moyens de modulation temporelle étant adaptés pour réduire la densité d'énergie déposée sur la surface à traiter.

De façon particulièrement avantageuse, le système laser selon la présente divulgation comporte un système optique adapté pour focaliser le faisceau laser sur une zone ayant une surface d'environ 0,01 millimètres carrés, c'est-à-dire une surface environ 1000 fois plus petite qu'avec un système laser pour traitement dermatologique selon l'art antérieur.

La figure 2 représente un système laser impulsionnel pour traitement dermatologique selon un premier mode de réalisation. Le système laser est basé sur une technologie de diode laser, laser à fibre, laser à solide, laser à colorant ou laser à gaz. Le système laser comporte un oscillateur 1, un amplificateur optique 2, un modulateur acousto-optique 3 et un générateur électrique 4. L'oscillateur 1 génère un faisceau d'impulsions lumineuses 10. L'amplificateur optique 2 reçoit le faisceau d'impulsions lumineuses 10 et génère un faisceau d'impulsions laser 20.

Le faisceau d'impulsions laser 20 a une longueur d'onde comprise généralement entre 700 nm et 10600 nm. La durée d des impulsions laser 20 délivrées par le système laser est comprise entre 500 fs et 100 ps, et de préférence inférieure à 50 ps. La première fréquence de répétition F₁ des impulsions laser délivrées est comprise entre 1 kHz et 10 GHz.

Le modulateur acousto-optique 3 est relié au générateur électrique 4. Plus précisément, le générateur électrique 4 est adapté pour générer un signal radio-fréquence 40 appliqué aux électrodes du modulateur acousto-optique 3. Le modulateur acousto-optique 3 reçoit le faisceau d'impulsions laser 20 et délivre un faisceau d'impulsions laser modulé temporellement 100.

Selon un premier exemple du premier mode de réalisation, le générateur électrique 4 est adapté pour générer un signal radio-fréquence 40 en forme de porte ou de créneau, ayant une durée T₃ supérieure à la première période de répétition T₁. La première période de répétition T₁ est égale à l'inverse de la première fréquence de répétition F_{1 :} T₁ = 1/ F₁.Par conséquent, le modulateur acousto-optique 3 sélectionne une pluralité d'impulsions laser formant un macro-pulse, aussi appelé paquet d'impulsions. Ainsi, le modulateur acousto-optique bloque les impulsions laser pendant une durée T₄ supérieure à la première période de répétition T₁. Cette modulation temporelle des impulsions laser permet de limiter la densité d'énergie déposée sur la surface à traiter. De préférence, la densité d'énergie ainsi déposée est inférieure à 0,01 J/cm² sur un spot focalisé ayant un diamètre compris entre 10 µm et 20 mm, et de préférence inférieur à ~1 mm.

La durée T₃ d'un macropulse est égale au produit du nombre N d'impulsions laser dans un macro-pulse par la première période de répétition T₁ des impulsions laser. Chaque impulsion laser du macro-pulse a une quantité d'énergie inférieure ou égale à 1 microjoule.

De façon optionnelle, le modulateur acousto-optique 3 est adapté pour générer séquentiellement plusieurs macro-pulses. Le modulateur acousto-optique peut être adapté pour générer une pluralité de macro-pulses avec une deuxième période de répétition T₂. La deuxième période de répétition T₂ est égale à la somme de la durée T₃ d'un créneau et de la durée T₄ entre deux créneaux consécutifs. Autrement dit, le signal radio-fréquence 40 est nul pendant la durée T₄. La sélection de la durée T₃ et de la durée T₄ permet de moduler la durée T₂ d'un macro-pulse et la fréquence de répétition des macro-pulses, notée ici deuxième fréquence de répétition F₂ = 1/T₂. De cette manière, le modulateur acousto-optique permet de réduire le nombre d'impulsions laser incidentes sur la surface à traiter et de moduler la densité d'énergie déposée.

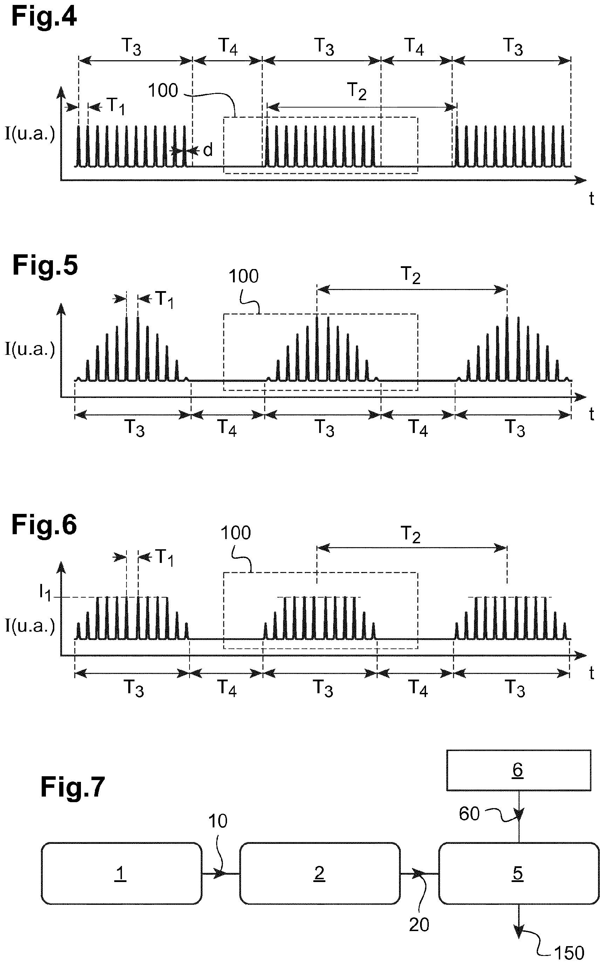

La figure 4 illustre, en fonction du temps t, l'intensité lumineuse du faisceau d'impulsions laser modulé temporellement 100 généré par un système laser illustré schématiquement sur la figure 2, dans lequel le générateur électrique 4 applique un signal radio-fréquence 40 en forme de créneau rectangulaire. Dans cet exemple, les impulsions lumineuses d'un macro-pulse ont toutes à peu près la même intensité lumineuse.

Selon une variante, le générateur électrique 4 produit un signal radio-fréquence 40 modulé en intensité pendant une durée T₃ et nul pendant une durée T₄. Plus précisément, pendant une durée T₃, le signal radio-fréquence 40 est croissant puis décroissant. Par exemple, le signal radio-fréquence 40 a une forme triangulaire pendant la durée T₃. De façon optionnelle, le signal radio-fréquence 40 est périodique avec une deuxième période de répétition T₂, égale à la somme de la durée T₃ et de la durée T₄. Comme illustré par exemple sur la figure 5, un tel signal radio-fréquence 40 appliqué au modulateur acousto-optique 3 permet de moduler l'intensité lumineuse des impulsions laser incidentes sur la surface à traiter non seulement en supprimant des impulsions laser en dehors du ou des macro-pulses, pendant la durée T₄, mais à l'intérieur de chaque macro-pulse. On obtient ainsi un macro-pulse contenant N impulsions laser, dans lequel l'intensité lumineuse des impulsions laser est croissante puis décroissante. Comme illustré sur la figure 5, ce macropulse peut être répété périodiquement à la deuxième période de répétition T₂, autrement dit avec la deuxième fréquence de répétition F_{2.}

Selon une autre variante, le générateur électrique 4 produit un signal radio-fréquence 40 modulé en intensité pendant une durée T₃ et nul pendant une durée T₄. Plus précisément dans cette autre variante, pendant la durée T₃, le signal radio-fréquence 40 est croissant puis constant puis décroissant. Par exemple, le signal radio-fréquence 40 a une forme trapézoïdale pendant la durée T₃. De façon optionnelle, le signal radio-fréquence 40 est périodique avec une deuxième période de répétition T₂, égale à la somme de la durée T₃ et de la durée T₄. Comme illustré par exemple sur la figure 6, un tel signal radio-fréquence 40 appliqué au modulateur acousto-optique 3 permet de moduler l'intensité lumineuse des impulsions laser incidentes sur la surface à traiter non seulement en supprimant des impulsions laser en dehors du ou des macro-pulses, pendant la durée T₄, mais à l'intérieur de chaque macro-pulse. On obtient ainsi un macro-pulse contenant N impulsions laser, dans lequel l'intensité lumineuse des impulsions laser est croissante, constante sur plusieurs impulsions successives puis décroissante. Comme illustré sur la figure 6, ce macropulse peut être répété périodiquement à la deuxième période de répétition T₂, autrement dit avec la deuxième fréquence de répétition F_{2.}

Selon un deuxième mode de réalisation, le système laser ne comporte pas de modulateur acousto-optique mais comporte un générateur électrique 14. L'amplificateur optique 2 comporte un dispositif de pompage optique 12, par exemple une ou plusieurs diodes laser monomodes ou multimodes, ou encore une ou plusieurs lampes flashs. Le générateur électrique 4 est relié à la source de courant-tension du dispositif de pompage optique. Plus précisément, le générateur électrique 14 est adapté pour générer un signal radio-fréquence 41 appliqué à la source de courant-tension du dispositif de pompage optique. L'amplificateur optique 2 reçoit le faisceau d'impulsions lumineuses 10 de l'oscillateur et génère un faisceau d'impulsions laser modulé temporellement 150. Ainsi, le générateur électrique 14 permet de moduler directement l'intensité lumineuse des impulsions amplifiées par l'amplificateur optique 2.

Selon un premier exemple de ce deuxième mode de réalisation, le générateur électrique 14 produit un signal radio-fréquence 41 modulé en intensité, par exemple de forme rectangulaire non nul pendant une durée T₃, le signal radio-fréquence 41 étant nul pendant une durée T₄. Ainsi, dans ce premier exemple, pendant la durée T₃, le signal radio-fréquence 41 est constant. De façon optionnelle, le signal radio-fréquence 41 est périodique avec une deuxième période de répétition T₂, égale à la somme de la durée T₃ et de la durée T₄. Comme illustré par exemple sur la figure 4, un tel signal radio-fréquence 41 appliqué à la source de courant-tension du dispositif de pompage optique 12 du système amplificateur optique 2 permet de moduler l'intensité lumineuse des impulsions laser amplifiées 100, en supprimant des impulsions laser en dehors du ou des macro-pulses. On obtient ainsi un macro-pulse contenant N impulsions laser, dans lequel l'intensité lumineuse des impulsions laser est constante sur les N impulsions successives. Comme illustré sur la figure 4, ce macropulse peut être répété périodiquement à la deuxième période de répétition T₂, autrement dit avec la deuxième fréquence de répétition F_{2.}

Selon une première variante de ce deuxième mode de réalisation, le générateur électrique 14 produit un signal radio-fréquence 41 modulé en intensité, par exemple de forme triangulaire pendant une durée T₃, le signal radio-fréquence 41 étant nul pendant une durée T₄. Ainsi, dans cette variante, pendant la durée T₃, le signal radio-fréquence 41 est croissant puis décroissant. De façon optionnelle, le signal radio-fréquence 41 est périodique avec une deuxième période de répétition T₂. Comme illustré par exemple sur la figure 5, un tel signal radio-fréquence 41 appliqué à la source de courant-tension du dispositif de pompage optique du système amplificateur optique permet de moduler l'intensité lumineuse des impulsions laser amplifiées à l'intérieur d'un macro-pulse. On obtient ainsi un macro-pulse contenant N impulsions laser, dans lequel l'intensité lumineuse des impulsions laser est croissante puis décroissante. Comme illustré sur la figure 5, ce macropulse peut être répété périodiquement à la deuxième période de répétition T₂, autrement dit avec la deuxième fréquence de répétition F_{2.}

Selon une autre variante de ce deuxième mode de réalisation, le générateur électrique 14 produit un signal radio-fréquence 41 41 modulé en intensité, par exemple de forme trapézoïdale pendant une durée T₃, le signal radio-fréquence 41 étant nul pendant une durée T₄. Ainsi, dans cette variante, pendant la durée T₃, le signal radio-fréquence 41 est croissant puis décroissant. De façon optionnelle, le signal radio-fréquence 41 est périodique avec une deuxième période de répétition T₂. Comme illustré par exemple sur la figure 5, un tel signal radio-fréquence 41 appliqué à la source de courant-tension du dispositif de pompage optique du système amplificateur optique permet de moduler l'intensité lumineuse des impulsions laser amplifiées à l'intérieur d'un macro-pulse. On obtient ainsi un macro-pulse contenant N impulsions laser, dans lequel l'intensité lumineuse des impulsions laser est croissante, constante sur plusieurs impulsions successives puis décroissante. Comme illustré sur la figure 6, ce macropulse peut être répété périodiquement à la deuxième période de répétition T₂, autrement dit avec la deuxième fréquence de répétition F_{2.}

L'homme du métier adaptera aisément la forme et les durées T₃ et T₄ du signal radio-fréquence 40 ou 41 pour obtenir la modulation d'intensité lumineuse des impulsions laser en fonction de l'application et du traitement dermatologique considéré.

Ce deuxième mode de réalisation permet de moduler le signal de pompe de l'amplificateur optique pour sélectionner une pluralité d'impulsions laser formant un macro-pulse, de manière à limiter la densité d'énergie déposée par le faisceau laser à moins de 0,01 J/cm².

Du fait de l'énergie limitée du faisceau d'impulsions laser, la présente divulgation permet de focaliser le faisceau laser sur une surface plus petite. Le spot est ainsi 1000 fois plus petit qu'un spot laser généré par un système laser utilisé en dermatologie de l'art antérieur. Au lieu de couvrir une surface de quelques millimètres carrés, le faisceau laser couvre alors seulement une surface d'environ 0,01 millimètres carrés, soit un faisceau laser focalisé sur un disque ayant un diamètre d'environ 50 à 100 microns. Cependant, certains traitements dermatologiques doivent être effectués sur de grandes surfaces, de plusieurs centimètres carrés à quelques dizaines de centimètres carrés. L'utilisation d'un spot laser ayant un diamètre inférieur à environ un dixième de mm peut prendre énormément de temps ce qui est a priori dissuasif.

Toutefois, on utilise selon la présente divulgation une première fréquence de répétition F₁ qui est 10000 à 100000 fois plus élevée que la fréquence de répétition F d'un système laser de l'art antérieur. L'utilisation d'un système laser à très haute fréquence de répétition F₁ permet d'obtenir un effet cumulatif sur la surface traitée et d'induire un effet non-linéaire de photo-disruption. Autrement dit, le système laser de la présente divulgation opère dans un nouveau régime. En effet, dans ce nouveau régime, chaque impulsion individuelle n'a pas l'énergie ou l'intensité lumineuse suffisante pour produire des ruptures optiques de type photo-disruption. Cependant, grâce aux processus non-linéaires, les interactions cumulatives d'une pluralité d'impulsions laser à la première fréquence de répétition F₁ induisent des changements structurels dans les matériaux entraînant une amélioration de l'absorption. Après un nombre déterminé d'impulsions, qui dépend de la surface à traiter et d'un seuil d'intensité lumineuse, il se produit un phénomène de photo-disruption. L'intérêt majeur de ce nouvel effet est lié à ce que la densité d'énergie déposée sur la peau est limitée et de préférence inférieure ou égale à 0.01 J/cm² ce qui permet de préserver la surface traitée en dermatologie de phénomènes d'ablation et d'échauffement indésirables.

L'interaction cumulative d'une série de N impulsions laser d'énergie microjoule à une première fréquence de répétition F₁ élevée permet d'obtenir un effet de photo-disruption sélective en fonction de la cible concernée, choisie de manière non limitative par exemple parmi des pigments endogènes, pigments exogènes, la mélanine ou le sébum.

Selon un troisième mode de réalisation, qui peut être mis en oeuvre séparément ou en combinaison avec le premier ou le deuxième mode de réalisation, le système laser comporte un oscillateur 1, un système amplificateur optique 2 et comporte en outre un dispositif de balayage de faisceau 5, aussi appelé scanner. Un système électrique 6 de commande et de synchronisation est relié au dispositif de balayage de faisceau 5. Le dispositif de balayage de faisceau 5 est placé en sortie de la chaîne laser, ici en sortie du système amplificateur optique 2. A titre d'exemple non limitatif, le dispositif de balayage de faisceau 5 comporte un scanner à deux axes transverses. Un tel dispositif de balayage de faisceau 5 permet de déplacer le faisceau laser suivant deux directions transverses sur la surface à traiter. Le système électrique 6 de commande et de synchronisation génère un signal 60 électrique ou électronique qui permet de commander la direction, le sens et la vitesse de déplacement sur chaque axe du dispositif de balayage de faisceau 5.

Le système électrique 6 de commande et de synchronisation et le dispositif de balayage de faisceau 5 sont configurés pour déplacer le faisceau laser sur la surface à traiter de manière à limiter la densité d'énergie déposée, de préférence à moins de 0,01 J/cm². En fonction de la vitesse de déplacement, il est ainsi possible de modifier la quantité d'énergie déposée par unité de surface pour un temps donné, autrement dit de moduler la densité d'énergie déposée sur la surface traitée.

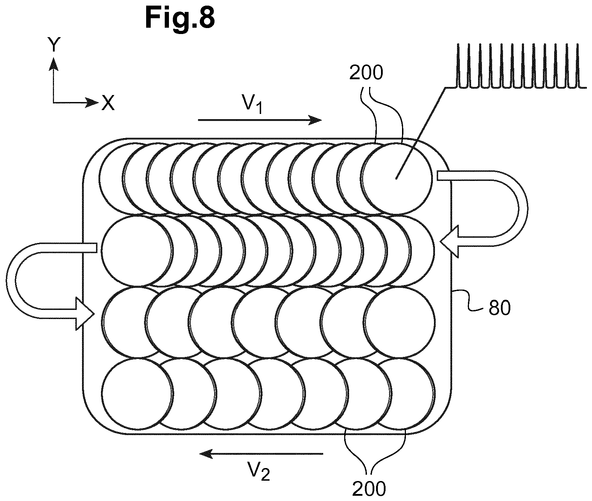

La figure 8 illustre un exemple de fonctionnement du système laser à impulsions combiné à un dispositif de balayage de faisceau 5. La figure 8 représente une surface à traiter 80. On a représenté un repère XY dans le plan de la surface à traiter 80. Cette surface à traiter 80 s'étend par exemple sur un carré de 1 mm de côté. Un système optique focalise le faisceau d'impulsions laser de faible énergie par pulse et à haute fréquence de répétition F₁ pour former un spot 200 dans la surface à traiter 80. Le spot 200 est généralement de forme circulaire et a un diamètre d'environ 500 µm. Le système optique de focalisation peut être disposé en amont ou en aval du dispositif de balayage de faisceau 5.

On commence par exemple le traitement en appliquant un spot laser 200 en haut à gauche de la surface à traiter 80. Le dispositif de balayage de faisceau 5 déplace le faisceau laser suivant l'axe X avec une première vitesse de déplacement V1. Arrivé à proximité du bord de la surface à traiter 80, le dispositif de balayage de faisceau 5 applique un déplacement suivant la direction Y et change de sens suivant la direction X tout en conservant la première vitesse de déplacement V1. Arrivé à proximité de l'autre bord de la surface à traiter 80, le dispositif de balayage de faisceau 5 applique un déplacement suivant la direction Y et change de sens suivant la direction X tout en appliquant une deuxième vitesse de déplacement V2 supérieure à V1. Arrivé à proximité du bord de la surface à traiter 80, le dispositif de balayage de faisceau 5 applique un déplacement suivant la direction Y et change de sens suivant la direction X tout en conservant la deuxième vitesse de déplacement V2. Les vitesses de déplacement peuvent atteindre 8 m/s, ce qui permet de couvrir la surface à traiter 80 de 1 cm ² pour un spot de 1 mm en ~13 ms.

La vitesse de déplacement détermine le nombre d'impulsions accumulées à haute cadence et permet ainsi déterminer la quantité d'énergie déposée par unité de surface pour un temps donné. Ainsi, la densité d'énergie déposée est supérieure dans la zone où le déplacement du faisceau se fait à la vitesse V1, par comparaison à la zone de la surface à traiter où le déplacement du faisceau se fait à la vitesse V2.

La vitesse de déplacement suivant un axe X ou Y est généralement comprise entre 0,1m/s et 10 m/s et de préférence supérieure à 5 m/s.

Le déplacement du faisceau laser peut être continu ou être effectué pas par pas.

Le troisième mode de réalisation permet de moduler spatialement la densité d'énergie d'un faisceau d'impulsions laser faiblement énergétiques ayant une période de répétition F₁ élevée, de manière à réduire fortement la densité d'énergie déposée. Ce troisième mode de réalisation permet ainsi d'atteindre le régime d'interactions non-linéaires de photodisruption cumulative.

Selon un mode de réalisation particulier, le système laser combine une modulation temporelle des impulsions laser en macro-pulses comme décrit en lien avec le premier ou le deuxième mode de réalisation et un déplacement du spot laser sur la zone à traiter comme décrit en lien avec le troisième mode de réalisation. Cette combinaison d'une modulation temporelle et spatiale du faisceau d'impulsions laser permet d'accroitre la dynamique de réglage de la densité d'énergie déposée. Elle permet aussi d'améliorer la résolution spatiale du faisceau laser sans augmenter la durée totale du traitement laser pour une surface donnée.

Un seul système laser combinant un modulateur acousto-optique et un dispositif de balayage de faisceau 5 permet de moduler à la fois temporellement et spatialement la densité d'énergie des impulsions laser à haute fréquence de répétition et de faible énergie sur une dynamique très étendue.

De manière encore plus simple, un seul système laser combinant un système amplificateur optique et un dispositif de balayage de faisceau 5, dans lequel un générateur électrique de signal RF est relié à la source de courant-tension du dispositif de pompage optique du système amplificateur optique permet de moduler à la fois temporellement et spatialement la densité d'énergie des impulsions laser à haute fréquence de répétition et de faible énergie sur une dynamique très étendue.

Un seul système laser combinant le premier et le troisième mode de réalisation ou le deuxième et le troisième mode de réalisation permet ainsi d'effectuer une gamme variée de traitements dermatologiques, qui nécessitaient auparavant plusieurs systèmes laser.

Le système laser selon la présente divulgation opère dans un nouveau régime d'interaction, ici appelé de photo-disruption cumulative des tissus, qui repose sur l'utilisation d'un nombre N d'impulsions laser, de préférence ultra-brèves, faiblement énergétiques, à haute fréquence de répétition, et sur une modulation temporelle et/ou spatiale du faisceau laser pour limiter la densité d'énergie déposée sur la surface à traiter.

L'invention permet de focaliser le faisceau sur un spot de très petite taille ce qui permet un meilleur ciblage de la zone à traiter. La couverture spatiale de la zone à traiter est obtenue en déplaçant le faisceau laser, de préférence au moyen d'un dispositif de balayage de faisceau. Ce balayage du faisceau peut être ajusté en fonction des contours de la surface de manière à appliquer le faisceau laser sur toute la surface à traiter 80 sans déborder autour de cette surface à traiter. L'invention est définie dans les revendications suivantes.

## Revendications

1. Système laser impulsionnel pour traitement dermatologique, le système laser impulsionnel comprenant une source lumineuse (1) adaptée pour émettre un faisceau d'impulsions lumineuses (10) et un système amplificateur optique (2) adapté pour recevoir le faisceau d'impulsions lumineuses (10) et pour générer un faisceau d'impulsions laser (20) à une première fréquence de répétition (F₁), la durée (d) d'une impulsion laser (20) est comprise entre 100 femtoseconde et 100 picosecondes, la première fréquence de répétition (F₁) est comprise entre 1 kHz et 10 GHz, chaque impulsion laser ayant une quantité d'énergie inférieure ou égale à 1 microjoule, et
**caractérisé en ce que** :
le système laser impulsionnel comporte en outre des moyens de modulation temporelle du faisceau d'impulsions laser (20), ces moyens de modulation temporelle étant adaptés pour réduire la densité d'énergie déposée sur une surface à traiter et pour générer une densité d'énergie comprise entre 0.0001 J/cm² et 0.01 J/cm², dans lequel les moyens de modulation temporelle du faisceau d'impulsions laser (20) comprennent un modulateur acousto-optique (3) et un générateur électrique (4), le modulateur acousto-optique (3) étant disposé en sortie de système amplificateur optique (2), le générateur électrique (4) étant adapté pour générer un signal radio-fréquence (40) appliqué aux électrodes du modulateur acousto-optique (3), le signal radio-fréquence (40) étant adapté pour que le modulateur acousto-optique (3) sélectionne un paquet (100) d'impulsions laser ou dans lequel le système amplificateur optique (2) comporte un dispositif de pompage optique (12) et une source de courant-tension et dans lequel les moyens de modulation temporelle du faisceau d'impulsions laser (20) comprennent un générateur électrique (14) adapté pour générer un signal radio-fréquence (41) appliqué à la source de courant-tension du dispositif de pompage optique (12), le signal radio-fréquence (41) étant adapté pour que le système amplificateur optique (2) génère un paquet (100) d'impulsions laser.

2. Système selon la revendication 1, dans lequel le générateur électrique (4, 14) est en outre adapté pour moduler temporellement le signal radio-fréquence (40, 41) de manière à moduler en intensité les impulsions laser d'un paquet (100) d'impulsions laser.

3. Système selon l'une des revendications 1 à 2, dans lequel un paquet (100) d'impulsions laser comporte un nombre (N) d'impulsions laser compris entre 100 et 1000000.

4. Système selon l'une des revendications 1 à 3, comportant en outre des moyens de modulation spatiale du faisceau d'impulsions laser (20), ces moyens de modulation spatiale étant adaptés pour réduire la densité d'énergie déposée sur une surface à traiter et pour générer une densité d'énergie comprise entre 0.0001 J/cm² et 0.01 J/cm², dans lequel les moyens de modulation spatiale du faisceau d'impulsions laser (20) comprennent un dispositif de balayage de faisceau (5), le dispositif de balayage de faisceau (5) étant adapté pour déplacer le faisceau d'impulsions laser (20) sur une région d'intérêt de la surface à traiter de manière à limiter la densité d'énergie déposée dans la région d'intérêt.

5. Système selon la revendication 4, dans lequel le dispositif de balayage de faisceau (5) est adapté pour déplacer le faisceau laser suivant deux axes transverses.

6. Système selon la revendication 4 ou la revendication 5, dans lequel la vitesse de déplacement du faisceau lumineux dans la région d'intérêt est comprise entre 0,1m/s et 10 m/s.

7. Système selon l'une des revendications 1 à 6 comportant en outre un système optique de focalisation, le système optique de focalisation étant adapté pour focaliser le faisceau laser sur un spot ayant un diamètre inférieur à environ 20 mm.

8. Système selon l'une des revendications 1 à 7, dans lequel la source lumineuse (1) et le système amplificateur optique (2) sont adaptés pour émettre le faisceau d'impulsions laser (20) à une longueur d'onde comprise entre 700 nm et 10600 nm.

## Patentansprüche

1. Gepulstes Lasersystem für dermatologische Behandlungen, wobei das gepulste Lasersystem eine zum Aussenden eines Strahls von Leuchtimpulsen (10) ausgelegte Lichtquelle (1) und ein zum Empfangen des Strahls von Leuchtimpulsen (10) und zum Erzeugen eines Strahls von Laserimpulsen (20) mit einer ersten Wiederholungsfrequenz (Fi) ausgelegtes optisches Verstärkersystem (2) aufweist, wobei die Dauer (d) eines Laserimpulses (20) zwischen 100 Femtosekunden und 100 Picosekunden beträgt, die erste Wiederholungsfrequenz (Fi) zwischen 1 kHz und 10 GHz beträgt, wobei jeder Laserimpuls eine Energie von weniger als oder gleich 1 Mikrojoule hat, und
**dadurch gekennzeichnet, daß**
das gepulste Lasersystem außerdem Mittel für die zeitliche Modulation des Strahls von Laserimpulsen (20) aufweist, wobei die Mittel für die zeitliche Modulation dazu ausgelegt sind, die auf eine zu behandelnde Fläche aufgebrachte Energie zu reduzieren und eine Energiedichte zwischen 0,0001 J/cm² und 0,01 J/cm² zu erzeugen, wobei die Mittel für die zeitliche Modulation des Strahls von Laserimpulsen (20) einen akusto-optischen Modulator (3) und einen elektrischen Generator (4) aufweisen, wobei der akusto-optische Modulator (3) am Ausgang des optischen Verstärkersystems (2) angeordnet ist, wobei der elektrische Generator (4) dazu ausgelegt ist, ein Radiofrequenzsignal (40) zu erzeugen, das an die Elektroden des akusto-optischen Modulators (3) angelegt wird, wobei das Radiofrequenzsignal (40) so angepaßt ist, daß der akusto-optische Modulator (3) ein Paket Laserimpulse (100) auswählt, oder wobei das optische Verstärkersystem (2) eine optische Pumpvorrichtung (12) und eine Strom-Spannungs-Quelle aufweist und wobei die Mittel für die zeitliche Modulation des Strahls von Laserimpulsen (20) einen elektrischen Generator (14) aufweisen, der dazu ausgelegt ist, ein Radiofrequenzsignal (41) zu erzeugen, das an die Strom-Spannungs-Quelle der optischen Pumpvorrichtung (12) angelegt wird, wobei das Radiofrequenzsignal (41) so angepaßt ist, daß das optische Verstärkersystem (2) ein Paket Laserimpulse (100) erzeugt.

2. System gemäß Anspruch 1, bei dem der elektrische Generator (4, 14) außerdem dazu ausgelegt ist, das Radiofrequenzsignal (40, 41) zeitlich so zu modulieren, daß die Laserimpulse eines Pakets Laserimpulse (100) hinsichtlich ihrer Stärke moduliert werden.

3. System gemäß einem der Ansprüche 1 bis 2, bei dem ein Paket Laserimpulse (100) eine Anzahl (N) Laserimpulse zwischen 100 und 1000000 aufweist.

4. System gemäß einem der Ansprüche 1 bis 3, das außerdem Mittel für eine räumliche Modulation des Strahls Laserimpulse (20) aufweist, wobei diese Mittel für eine räumliche Modulation dazu ausgelegt sind, die auf eine zu behandelnde Fläche aufgebrachte Energie zu reduzieren und eine Energiedichte zwischen 0,0001 J/cm² und 0,01 J/cm² zu erzeugen, wobei die Mittel für eine räumliche Modulation des Strahls Laserimpulse (20) eine Strahlabtastvorrichtung (5) aufweisen, wobei die Strahlabtastvorrichtung (5) dazu ausgelegt ist, den Strahl Laserimpulse (20) auf einem Zielgebiet der zu behandelnden Fläche zu bewegen, um die auf das Zielgebiet aufgebrachte Energie zu begrenzen.

5. System gemäß Anspruch 4, bei dem die Strahlabtastvorrichtung (5) dazu ausgellegt ist, den Laserstrahl entlang zweier Querachsen zu bewegen.

6. System gemäß Anspruch 4 oder Anspruch 5, bei dem die Bewegungsgeschwindigkeit des Lichtstrahls im Zielgebiet zwischen 0,1 m/s und 10 m/s beträgt.

7. System gemäß einem der Ansprüche 1 bis 6, das außerdem ein optisches Fokussierungssystem aufweist, wobei das optische Fokussierungssystem dazu ausgelegt ist, den Laserstrahl auf einem Punkt zu fokussieren, der einen Durchmesser von weniger als ungefähr 20 mm hat.

8. System gemäß einem der Ansprüche 1 bis 7, bei dem die Lichtquelle (1) und das optische Verstärkersystem (2) dazu ausgelegt sind, den Strahl Laserimpulse (20) mit einer Wellenlänge zwischen 700 nm und 10600 nm auszusenden.

## Claims

1. A pulsed laser system for dermatological treatment, the pulsed laser system comprising a light source (1) suitable for emitting a light pulse beam (10) and an optical amplifier system (2) suitable for receiving the light pulse beam (10) and generating a laser pulse beam (20) at a first repetition frequency (F₁), the duration (d) of a laser pulse (20) is between 100 femtoseconds and 100 picoseconds, the first repetition frequency (Fi) is between 1 kHz and 10 GHz, each laser pulse having a quantity of energy lower than or equal to 1 microjoule, and **characterized in that**:
the pulsed laser system further includes means for temporally modulating the laser pulse beam (20), these temporal modulation means being adapted to reduce the density of energy deposited on a surface area to be treated and to generate a density of energy between 0.0001 J/cm² and 0.01 J/cm², wherein the means for temporally modulating the laser pulse beam (20) comprise an acousto-optic modulator (3) and an electric generator (4), the acousto-optic modulator (3) being arranged at the output of the optical amplifier system (2), the electric generator (4) being adapted to generate a radiofrequency signal (40) applied to the electrodes of the acousto-optic modulator (3), the radiofrequency signal (40) being adapted so that the acousto-optic modulator (3) selects a burst (100) of laser pulses or wherein the optical amplifier system (2) includes an optical pumping device (12) and a current-voltage source, and wherein the means for temporally modulating the laser pulse beam (20) comprise an electric generator (14) adapted to generate a radiofrequency signal (41) applied to the current-voltage source of the optical pumping device (12), the radiofrequency signal (41) being adapted so that the optical amplifier system (2) generates a burst (100) of laser pulses.

2. The system according to claim 1, wherein the electric generator (4, 14) is further adapted to temporally modulate the radiofrequency signal (40, 41) in such a way as to modulate in intensity the laser pulses of a burst (100) of laser pulses.

3. The system according to any one of claims 1 to 2, wherein a burst (100) of laser pulses includes a number (N) of laser pulses between 100 and 1,000,000.

4. The system according to any one of claims 1 to 3, further comprising means for spatially modulating the laser pulse beam (20), these means for spatially modulating the laser pulse beam (20) being adapted to reduce the density of energy deposited on a surface area to be treated and to generate a density of energy between 0.0001 J/cm² and 0.01 J/cm², wherein the means for spatially modulating the laser pulse beam (20) comprise a beam scanning device (5), the beam scanning device (5) being adapted to move the laser pulse beam (20) on a region of interest of the surface area to be treated in such a way as to limit the density of energy deposited in the region of interest.

5. The system according to claim 4, wherein the beam scanning device (5) is adapted to move the laser beam along two transverse axes.

6. The system according to claim 4 or claim 5, wherein the moving speed of the light beam in the region of interest is between 0.1 m/s and 10 m/s.

7. The system according to any one of claims 1 to 6, further comprising an optical focusing system, the optical focusing system being adapted to focus the laser beam to a spot having a diameter lower than about 20 mm.

8. The system according to any one of claims 1 to 7, wherein the light source (1) and the optical amplifier system (2) are adapted to emit the laser pulse beam (20) at a wavelength between 700 nm and 10,600 nm.
